(19) Europäisches Patentamt
European Patent Office
Office européen des brevets

(11) **EP 2 183 124 B1**

(12) **EUROPEAN PATENT SPECIFICATION**

(45) Date of publication and mention
of the grant of the patent:
**19.12.2012 Bulletin 2012/51**

(51) Int Cl.:
**B60K 28/06** *(2006.01)* **G01N 33/497** *(2006.01)*

(21) Application number: **08789508.2**

(22) Date of filing: **31.07.2008**

(86) International application number:
**PCT/IB2008/053077**

(87) International publication number:
**WO 2009/019637 (12.02.2009 Gazette 2009/07)**

(54) **ELECTRONIC DEVICE FOR PREVENTION AND CONTROL OF THE STREET ACCIDENTS RISKS ATTRIBUTABLE TO THE USE OF ALCOHOL AND/OR THEIR ASSOCIATED SUBSTANCES**

ELEKTRONISCHE VORRICHTUNG ZUR VORBEUGUNG UND KONTROLLE VON VERKEHRSUNFALLSRISIKO AUFGRUND VON ALOKOHOL UND/ODER DAMIT ASSOZIIERTEN SUBSTANZEN

DISPOSITIF ÉLECTRONIQUE POUR LA PRÉVENTION ET LA MAÎTRISE DES RISQUES D'ACCIDENTS DE LA ROUTE ATTRIBUABLES À L'USAGE D'ALCOOL ET/OU DE SUBSTANCES ASSOCIÉES

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **03.08.2007 IT PD20070075 U**
**31.10.2007 IT PD20070359**

(43) Date of publication of application:
**12.05.2010 Bulletin 2010/19**

(73) Proprietor: **Angel S.r.l.**
**80128 Napoli (IT)**

(72) Inventors:
• **AZZENA, Gianfranco**
**I-44100 Ferrara (IT)**
• **LA GATTA, Antonio**
**I-35037 Teolo (pd) (IT)**

(74) Representative: **Vinci, Marcello**
**Ufficio Veneto Brevetti**
**Via Sorio 116**
**35141 Padova (IT)**

(56) References cited:
EP-A- 1 441 212    WO-A-2007/046745
GB-A- 2 232 284    US-A1- 2005 099 310

**Description**

DESCRIPTION

The electronic device's observance and final data processing to the prevention and control of the street accidents risks attributable to the use of alcohol and their associated substances is as a principle scope to the diminution and the prevention of the street accidents determined from the alcohol and/or their associated substance consumption. Concerning the associated substances, it means to all substances that can alter the conduction capacity of a vehicle such as drug substances, medicine, etc. The aforementioned problem is quite increasing in the deathly street accidents determined from the alcohol and/or associated substances. Presently, the available system to measure the alcohol level that has been used by drivers is breathalyzers.

These tools are essential subjects to 2 problems:

● First problem is given to the fact that the subject must be volunteer to be tested by the tool.
● Second is the lack of active feedback system from these tools.

EP 1 441 212 discloses an ethanol sensing system operating to collect a representative sample of ethanol from the breath of a driver within the passenger compartment of a vehicle.

The actual scope is to accomplish a system able to point out the presence of alcohol and/or associated substances without direct willing come from the subject that allows an alteration to characteristic driving performances.

It is well known that the presence of alcohol in the blood can be traced with analyzing the gases residue in the subject's breath. Particularly, the alcohol molecule and of other associated substances, being volatile through alveolus giving a fact to their presence concentration in the expiratory.

For this purpose, the new electronic device's observance and final data processing to the prevention and control of the street accidents attributable to the use of alcohol and their associated substances bases its real function on the gases residue accumulation around the driver's adjacent.

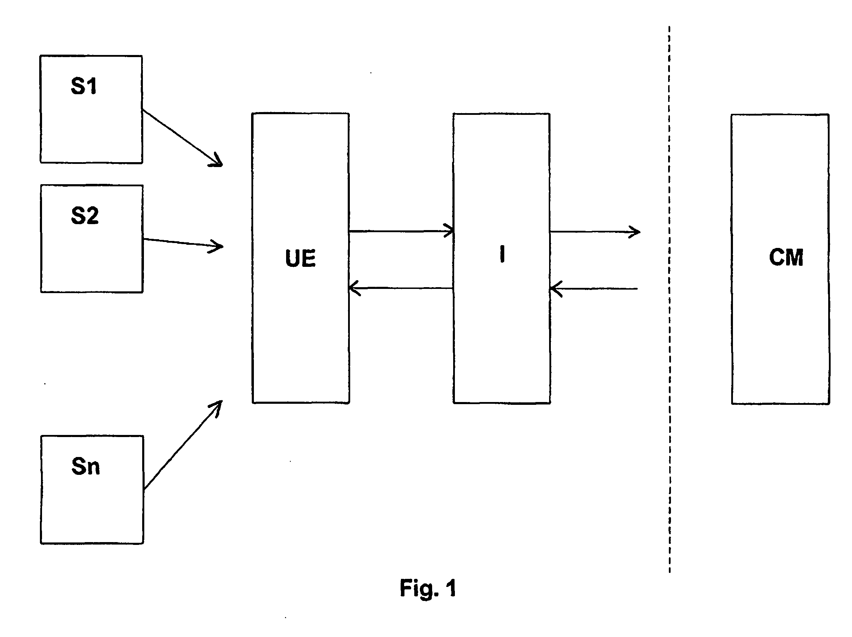

The enclosed table (fig.1) presents a scheme of brainwave operation as an illustrative subject but not limitative.

The electronic device according to the invention

For prevention and control of the street accidents risks attributable to the use of alcohol and their associated substance comprises the features of claim1.

The new electronic device according to the invention is based on the necessity of 3 different advanced functions:

- TRIANGULATION
- TEST OF COMBINED GAS
- NEGATIVE LOGICAL SYSTEM

TRIANGULATION: the mentioned processing unit (UE) obtains cyclical mentioned sensors (S1 ... SN), a proportional value to the alcohol and/or their associated substances' concentration, afterward defined as generic substance (X).

The obtained information speed is not critical and in each case, it must have a value not more than several minutes. To have a good processing algorithm, it is better to have an obtainable information speed in each 100 milliseconds.

Having more sensors (S1, ..., Sn) grants the system other unique abilities in distinguishing substances sources (X) position.

This distinction ability is absolutely necessary as there might be more person inside the car but only the emission of substance (X) came from the driver has the interest to the specific problem.

The information came from the mentioned sensors (S1, ..., Sn) are inserted in the mathematic pattern "A" dependent from the sensors arrangement (S1, ..., Sn), from their sensitivity, from the cabin form, from the presence laminar flows.

The systematic solution of "A" equation, being theoretically practicable, it is actually an impossible fact to resolve; therefore, it will use a "reverse founding" method to define a numerical equivalent.

The operating sequence of Triangulation is described as following:

1. The sensors information (S1, ..., Sn) and the internal sources of referred cabin arrange in normal function. In the moment that there is an indication of alcohol use from the subject, it will prepare the equivalent solution, for example using a manikin and come out from its mouth a concentration of alcohol defined as Xa (as a pattern). This (Xa) is a minimum value that being known in the law.

2. It sends a registered information from the sensors (S 1 ... Sn) and at the same time, the source will emit the composed gas from the air together with the substance with the pattern (Xa).

3. The traces comes from variety sensors (S1, ..., Sn), defines as S1r(t) ... Snr(t) registered from the internal "n" record.

4. After a moment of "tf" the registration can be interrupted. The moment of "tf" is relative to the cabin dimension, to the substance (X), to the available sensors (S1, ..., Sn). However, it is a value that cannot be more than 30 minutes, less than 180 seconds.

Therefore, the equation of "A" is a result of sequence mathematic operation in this exact order.

In the normal function, the sensors (S1 ... Sn) transfer continuously the information of the mentioned processing unit (UE).

It will insert a vector of S1(t) ... Sn(t) inside the processing unit (UE). The equation "A" implied in the same processing unit (UE) following "n" borrowing convolution between S1(t) and S1r(t) and then between S2(t) and S2r(t) until Sn(t) and Snr(t), according to the equation (1) as following;

$$\text{Equation (1)}$$

$$S1C(nt) = \sum_{k=0}^{n} S1(kt) * S1r((n-k)t)$$

At this point, there will be n vector available denominate S1c(t) ... Snc(t). Afterwards, it will follow time integration to determine a normal area subordinate to the equation (2).

$$\text{Equation (2)}$$

$$A1 = \sum_{n=0}^{n-max} S1C(nt)$$

$$A2 = \sum_{n=0}^{n-max} S2C(nt)$$

The result will be examined continuously with "n" coefficient (Ari) determined experimentally that typically can be posted around 30% of maximum possibility to the equation (2).

If a number is statistically significant (for example more than 50%) compare to the positive outcome, it can be considered as an alarm event that will trigger the sequent operation of the processing unit.

The possible intervention modes of the processing unit (UE) following an alarm event can be as following:

● Maximum car speed diminution interfering directly on the central motor interface.
● Acoustic and/or visual signal

COMBINATION OBSERVANCE OF MORE SUBSTANCES: In particular cases, it can be useful to monitor the presence and the concentration of gases, particularly alcohol and associated substances or gas and the oxygen concentration. For instance, a simultaneously high concentration of alcohol in the low oxygen concentration can be dangerous. Whenever it is necessary to monitor simultaneously more substances, the previous described system, the triangulation, can be used with different method that we would like to explain in the following.

The previous equation (1) and (2) is not a valid method approach but it is inserted as multiply constant of S1 (t) ... Sn(t) vector, a coefficient k1 ... kn according to the following criteria;

- Constant "kn" is more dangerous than the substances. It presume, for example, "kn=1" for the alcohol and to define that the danger of other substances "normal" according to the equation (3) based on the danger of alcohol taken.

### Equation (3)

$$Kns = \frac{\text{Other danger}}{\text{Alcohol danger}}$$

- Constant "kn" is taken as negative signal if the substance to be controlled is a substance that triggers an alarm event when its concentration is low and not high (for example the oxygen)

It is only at the aforementioned example, we can say typically, but not necessarily use.

"kn = 1" for the alcohol and "kn = -50" for the oxygen, please pay attention to the presence and position of less signal. Appropriate value can be experimentally deduced.

INTERVENTION SYSTEM: Particular attention is placed to the intervention system in the following alarm event test.

When the central motor (CM) has already all security systems attached to maintain the effectiveness of speed automatic mechanism, it is sufficient to send the aforementioned information of active alarm event and the maximum speed management must be conducted from the said processing unit (UE) inside your device.

An example of sequence algorithm that implements this function is as following:

- Street Security System. This algorithm is written in Assembler for AVR micro control with CORE 8051.

```
SSS Algorithm
VAR:
Alarm_flag = 1 if it is verified an alarm condition
Pos_acc = accelerator pedal position
Pos_max = pedal position correspond to the maximum acceleration
Speed = car's immediate speed
COST:
If Alarm_flag = 1 AND Pos_acc < 0.1 Pos_max AND Speed < 50
THEN Speed_max = 50.
```

Otherwise, if the alarm event condition is verified, then the mentioned control unit (I), interfacing the said central electronic motor (CM), raises the accelerator pedal position according to the reported position of correspondent pedal position at maximum speed and car's gear to determine a limited value and if the mentioned position of the accelerator pedal is less than 10% of the pedal position that correspondence to the maximum acceleration and if the car's gear for the speed is less than the mentioned limited value, then the control unit (I) communicates to the electronic central motor to give a speed limit to the mentioned value.

Furthermore, it is foreseen that the mentioned electronic device is bounded to be a car's transferable mode, permanently connected to the central electronic motor (CM) and activate the car's ignition system. The motor ignition is conditioned to the relevant and verified information as described previously.

## Claims

1. Electronic device for observing and processing information relevant to the air composition inside the car's compartment in general, purposed for the prevention and the control of street accidents risks attributable to the use of alcohol and/or its associated substances **characterized by** :

   ■ more than one environment sensors (S1, ..., Sn) of the relevant concentration or subject in the air of one or more substances (X);
   ■ at least one processing unit (UE) comparing the information obtained from the sensors (S1, ..., Sn) with predetermined

wherein said processing unit (UE) operates, directly or indirectly, an alarm event signal in consequence to the positive comparison of the information from the sensors,

and wherein the mentioned sensors (S1, ..., Sn) are adjusted to the certain substance (X) by detecting, over a certain time interval (tf), a predefined and known value of the concentration or subject (Xa) of said substance (X) injected in the car's compartment, thus producing n vectors of reference values (S1r(t), ..., Snr(t)), where t varies from 0 to tf.

2.  Electronic device for observing and processing information for the car in general, according to claim 1, **characterized by** at least one control unit or interface (I) in communication with the processing unit (UE) and interfaced to the car motor's electronic unit (CM), to modify the performance of the car's characteristic just in case of alarm event.

3.  Electronic device for observing and processing information for the car in general, according to claim 2, **characterized by** the fact that the mentioned control unit (I) is interfaced to the car motor's electronic unit (CM), to prevent the car's motor ignition.

4.  Electronic device for observing and processing information for the car in general, according to claim 2, **characterized by** the fact that the mentioned control unit (I) is interfaced to the car motor's electronic unit to give a limit to the car's speed when it is in motion or in the beginning of motor ignition.

5.  Electronic device for observing and processing information for the car in general, according to claim 1, **characterized by** at least one control unit (I), communicating with the processing unit (UE) and interfaced to the car motor's electronic unit (CM) to activate acoustic and/or light signal just in case of alarm event.

6.  Electronic device for observing and processing information for the car in general, according to claims 1, 2, 3, 4, 5, **characterized by** the fact that the mentioned substance (X) is alcohol or ethanol or drug or associated substances.

7.  Electronic device for observing and processing information for the car in general, according to claims 1, 2, 3, 4, 5, **characterized by** the fact that the mentioned substance (X) is oxygen.

8.  Electronic device for observing and processing information for the car in general, according to claims 1, 2, 3, 4, 5, **characterized by** the fact that the mentioned substance (X) is a drug substance and/or a medical substance and/or another substance in acceptable dosage which allows the driver to run a car.

9.  Electronic device for observing and processing information for the car in general, according to any of the previous claims, **characterized by** the fact that the mentioned sensors (S1, ..., Sn) are of the solid state type, for instance MEMS, or combustible cells.

10. Electronic device for observing and processing information for the car in general, according to any of the previous claims, **characterized by** the fact that the mentioned sensors (S1, ..., Sn) take the mentioned information in a time interval less than 180 seconds.

11. Electronic device for observing and processing information for the car in general, according to any of the previous claims, **characterized by** the fact of comprising two or more of said sensors (S1, ..., Sn) distributed in the car's compartment surrounding the driver's place, at a certain distance and other determined positions in regard to the driver's place.

12. Electronic device for observing and processing information for the car in general, according to any of the previous claims, **characterized by** the fact that the mentioned time interval (tf) is proportional to car's compartment size, according to the substance(s) being observed and to the available sensor's space.

13. Electronic device for observing and processing information for the car in general, according to claim 12, **characterized by** the fact that mentioned time interval (tf) is less than 180 seconds.

14. Electronic device for observing and processing information for the car in general, according to claims 12, 13, **characterized by** the fact that:

    ■ in normal function, the said sensors (S1, ..., Sn) observe the concentration value of the certain substance (X), producing a plurality of vectors of detected values (S1(t), ..., Sn(t));

■ the said processing unit (UE) gathers these measured values vectors (S1(t), ..., Sn(t)) and reference value vectors (S1r(t), ..., Snr(t)) and carries out n mutual convolutions between each measured value (Si(t)) and the correspondent reference value (Sir(t)), producing n vectors of values (S1C(t), ..., SnC(t)), where t varies from 0 to tf and where

$$SiC\,(nt) = \sum_{k=0}^{n} Si(kt) * Sir((n-k)t)$$

with i that varies from 1 to n;

the processing unit (UE) carries out a time integration of each one of said values SiC(t), producing n values (Ai), one for each sensor Si, where

$$Ai = \sum_{n=0}^{n-max} SiC(nt)$$

with that varies from 1 to n;

the processing unit (UE) compares said n values (Ai) with the corresponding n reference values (Ari) determined experimentally,

and where the alarm event or the immediate or deferred stop of the motor takes place in the case where the comparison results to be positive for a determinate percentage of said values (Ai).

**15.** Electronic device for observing and processing information for the car in general, according to claim 14, **characterized by** the fact that each of the reference values (Ari) is equal to 30% of the maximum possible value of the corresponding value (Ai).

**16.** Electronic device for observing and processing information for the car in general, according to claims 14, 15, **characterized by** the fact that the alarm event or the immediate or deferred stop of the motor takes place in the case where the comparison results to be positive for a percentage higher than or equal to 50% of said values (Ai).

**17.** Electronic device for observing and processing information for the car in general, according to claim 16, characterized from the fact that the comparison is positive if:

■ Ai < Ari, for substance (X) in which the concentration must be superiorly limited, like ethanol or associated substance;

■ Ai > Ari, for substance (X) in which the concentration must be inferiorly limited, like oxygen.

**18.** Electronic device for observing and processing information for the car in general, according to claim 1, **characterized by** the fact that the processing unit (UE) multiplies each of the measured values of the vector (S1(t), ..., Sn(t)) by a constant value (Kn), where;

■ Kn = 1, for alcohol or ethanol;

■ Absolute value of Kn > 1, for substances more dangerous than alcohol;

■ Kn < 0, for substances in which the concentration must be inferiorly limited.

**19.** Electronic device for observing and processing information for the car in general, according to claim 4, **characterized by** the fact that if the condition of alarm event is verified, then the control unit (I), interfacing with the motor's electronic unit (CM), detects the accelerator position in relation to the maximum acceleration position and instant speed of the vehicle with respect to a given limit value, and wherein, if said detected position of the accelerator pedal is lower than 10% compared to the pedal position corresponding to the maximum acceleration and if the instant speed of the car is lower than said limit value, then the control unit (I) instructs said electronic unit (CM) of the motor so that it limits the maximum instant speed to said limit value.

20. Electronic device for observing and processing information for the car in general, according to claim 19, **characterized by** the fact that the limit value of the maximum instant speed is 50 km/h.

21. Electronic device for observing and processing information for the car in general, according to claim 2, **characterized by** the fact that it is removably constrained to the vehicle, permanently connected to said electronic unit (CM) of the motor and activated by the ignition control and wherein the motor ignition is conditioned to the detection and to the verification of the information and of the conditions according to the previous claims.

**Patentansprüche**

1. Elektronische Vorrichtung zur Überwachung und Verarbeitung von Informationen über die Luftzusammensetzung im Fahrzeuginnenraum im Allgemeinen, zum Zweck der Prävention und Kontrolle des Risikos von Verkehrsunfällen aufgrund des Gebrauchs von Alkohol und/oder von mit diesem assoziierten Produkten dienend, durch Folgendes gekennzeichnet:

    ■ Mehr als einen Umgebungssensor (S1, ..., Sn) der relevanten Konzentration oder des Titers in der Luft von einer oder mehrerer Substanzen (X);
    ■ Wenigstens eine Verarbeitungseinheit (UE), welche die von den Sensoren (S1, ..., Sn) erhaltenen Informationen mit vorgegebenen Bezugsgrenzwerten vergleicht,

    wobei die besagte Verarbeitungseinheit (UE) direkt oder indirekt infolge des positivem Auftretens der Informationen von den Sensoren ein Alarmereignissignal aktiviert,
    und wobei die besagten Sensoren (S1, ..., Sn) auf die gewisse Substanz (X) eingestellt sind durch Erfassung über ein gewisses Zeitintervall (tf) eines vorbestimmten und bekannten Werts der Konzentration oder des Titers (Xa) der besagten, in den Innenraum eingespritzten Substanz (X), und somit durch Erzeugung von x Bezugswertvektoren (S1r(t), ..., Snr(t)), wobei t von 0 bis tf variiert.

2. Elektronische Vorrichtung zur Überwachung und Verarbeitung von Informationen über das Fahrzeug im Allgemeinen gemäß Patentanspruch 1, **gekennzeichnet durch** wenigstens ein Steuergerät oder eine Schnittstelle (I) in Kommunikation mit der Verarbeitungseinheit (UE) und verbunden mit dem Steuergerät des Fahrzeugmotors (CM), um nur im Fall eines Alarmereignisses die Leistung der Fahrzeugmerkmale zu ändern.

3. Elektronische Vorrichtung zur Überwachung und Verarbeitung von Informationen über das Fahrzeug im Allgemeinen gemäß Patentanspruch 2, **dadurch gekennzeichnet, dass** das besagte Steuergerät (I) mit dem Steuergerät des Fahrzeugmotors (CM) verbunden ist, um die Zündung des Fahrzeugmotors zu verhindern.

4. Elektronische Vorrichtung zur Überwachung und Verarbeitung von Informationen über das Fahrzeug im Allgemeinen gemäß Patentanspruch 2, **dadurch gekennzeichnet, dass** das besagte Steuergerät (I) mit dem Steuergerät des Fahrzeugmotors verbunden ist, um die Geschwindigkeit des Fahrzeugs zu begrenzen, wenn es sich in Bewegung oder am Beginn der Motorzündphase befindet.

5. Elektronische Vorrichtung zur Überwachung und Verarbeitung von Informationen über das Fahrzeug im Allgemeinen gemäß Patentanspruch 1, **gekennzeichnet durch** wenigstens ein Steuergerät (I), das mit der Verarbeitungseinheit (UE) kommuniziert und mit dem Steuergerät des Fahrzeugmotors (CM) verbunden ist, um nur im Fall eines Alarmereignisses akustische und/oder Lichtsignale zu aktivieren.

6. Elektronische Vorrichtung zur Überwachung und Verarbeitung von Informationen über das Fahrzeug im Allgemeinen gemäß den Patentansprüchen 1, 2, 3, 4, 5, **dadurch gekennzeichnet, dass** die besagte Substanz (X) Alkohol oder Äthanol oder eine Droge oder eine damit assoziierte Substanz ist.

7. Elektronische Vorrichtung zur Überwachung und Verarbeitung von Informationen über das Fahrzeug im Allgemeinen gemäß den Patentansprüchen 1, 2, 3, 4, 5, **dadurch gekennzeichnet, dass** die besagte Substanz (X) Sauerstoff ist.

8. Elektronische Vorrichtung zur Überwachung und Verarbeitung von Informationen über das Fahrzeug im Allgemeinen

EP 2 183 124 B1

gemäß den Patentansprüchen 1, 2, 3, 4, 5, **dadurch gekennzeichnet, dass** die besagte Substanz (X) eine Drogensubstanz und/oder ein Arzneimittel und/oder eine andere Substanz in annehmbarer Dosierung ist, welche es dem Fahrer erlauben, ein Fahrzeug zu führen.

9. Elektronische Vorrichtung zur Überwachung und Verarbeitung von Informationen über das Fahrzeug im Allgemeinen gemäß eines jeden der vorstehenden Patentansprüche, **dadurch gekennzeichnet, dass** die besagten Sensoren (S1, ..., Sn) Solide-State-Sensoren sind, zum Beispiel MEMS, oder Brennstoffzellen.

10. Elektronische Vorrichtung zur Überwachung und Verarbeitung von Informationen über das Fahrzeug im Allgemeinen gemäß eines jeden der vorstehenden Patentansprüche, **dadurch gekennzeichnet, dass** die besagten Sensoren (S 1, ..., Sn) die besagten Informationen in einem Zeitintervall von weniger als 180 Sekunden erfassen.

11. Elektronische Vorrichtung zur Überwachung und Verarbeitung von Informationen über das Fahrzeug im Allgemeinen gemäß eines jeden der vorstehenden Patentansprüche, **dadurch gekennzeichnet, dass** sie zwei oder mehr im Fahrzeuginnenraum in Fahrerplatznähe verteilte Sensoren (S1, ..., Sn) umfasst, in einem gewissen Abstand und anderen, bestimmten Positionen bezüglich des Fahrerplatzes.

12. Elektronische Vorrichtung zur Überwachung und Verarbeitung von Informationen über das Fahrzeug im Allgemeinen gemäß eines jeden der vorstehenden Patentansprüche, **dadurch gekennzeichnet, dass** das besagte Zeitintervall (tf) proportional zur Größe des Fahrzeuginnenraums ist, gemäß der/den überwachten Substanz(en) und dem für den Sensor zur Verfügung stehenden Raum.

13. Elektronische Vorrichtung zur Überwachung und Verarbeitung von Informationen über das Fahrzeug im Allgemeinen gemäß Patentanspruch 12, **dadurch gekennzeichnet, dass** das besagte Zeitintervall (tf) weniger als 180 Sekunden beträgt.

14. Elektronische Vorrichtung zur Überwachung und Verarbeitung von Informationen über das Fahrzeug im Allgemeinen gemäß Patentansprüchen 12, 13, **dadurch gekennzeichnet, dass**:

■ im normalen Betrieb die besagten Sensoren (S1, ..., Sn) den Konzentrationswert der gewissen Substanz (X) überwachen und eine Vielzahl von Vektoren erfasster Werte (S1(t), ..., Sn(t)) bilden;
■ die besagte Verarbeitungseinheit (UE) diese gemessenen Wertevektoren (S1(t), ..., Sn(t)) und Bezugswertvektoren (S1r(t), ..., Snr(t)) sammelt und x wechselseitige Faltungen zwischen jedem gemessenen Wert (Si(t)) und dem entsprechenden Bezugswert (Sir(t)) ausführt und x Wertevektoren (S1C(t), ..., SnC(t)) bildet, wobei t von 0 bis tf variiert und wobei

$$SiC(nt) = \sum_{k=0}^{n} Si(kt) * Sir((n-k)t)$$

mit von 1 bis x variierendem i;
die Verarbeitungseinheit (UE) eine zeitliche Integration jedes der besagten Werte SiC(t) ausführt und x Werte (Ai) bildet, einen je Sensor Si, wobei

$$Ai = \sum_{n=0}^{n-max} SiC(nt)$$

mit von 1 bis n variierendem i;
die Verarbeitungseinheit (UE) die besagten x Werte (Ai) mit den x entsprechenden, experimentell bestimmten Bezugswerten (Ari) vergleicht,

und wobei das Alarmereignis oder der unmittelbare oder verzögerte Motorstopp in dem Fall eintritt, in dem der

8

Vergleich für einen bestimmten Prozentsatz der besagten Werte (Ai) positiv resultiert.

**15.** Elektronische Vorrichtung zur Überwachung und Verarbeitung von Informationen über das Fahrzeug im Allgemeinen gemäß Patentanspruch 14, **dadurch gekennzeichnet, dass** jeder der Bezugswerte (Ari) gleich 30 % des maximal möglichen Werts des entsprechenden Werts (Ai) ist.

**16.** Elektronische Vorrichtung zur Überwachung und Verarbeitung von Informationen über das Fahrzeug im Allgemeinen gemäß Patentansprüchen 14, 15, **dadurch gekennzeichnet, dass** das Alarmereignis oder der unmittelbare oder verzögerte Motorstopp stattfindet, wenn der Vergleich für mehr oder gleich 50 % der besagten Werte (Ai) positiv resultiert.

**17.** Elektronische Vorrichtung zur Überwachung und Verarbeitung von Informationen über das Fahrzeug im Allgemeinen gemäß Patentanspruch 16, **dadurch gekennzeichnet, dass** der Vergleich dann positiv ist, wenn:

■ Ai < Ari für die Substanz (X), deren Konzentration nach oben begrenzt sein muss, wie Äthanol oder eine assoziierte Substanz;
■ Ai > Ari, für die Substanz (X), deren Konzentration nach unten begrenzt sein muss, wie Sauerstoff.

**18.** Elektronische Vorrichtung zur Überwachung und Verarbeitung von Informationen über das Fahrzeug im Allgemeinen gemäß Patentanspruch 1, **dadurch gekennzeichnet, dass** die Verarbeitungseinheit (UE) jeden der gemessenen Werte des Vektors (S1(t), ..., Sn(t)) mit einem konstanten Wert (Kn) multipliziert, wobei;

■ Kn = 1, für Alkohol oder Äthanol;
■ Absoluter Wert von Kn > 1, für gefährlichere Substanzen als Alkohol;
■ Kn < 0, für Substanzen, in denen die Konzentration nach unten begrenzt sein muss.

**19.** Elektronische Vorrichtung zur Überwachung und Verarbeitung von Informationen über das Fahrzeug im Allgemeinen gemäß Patentanspruch 4, **dadurch gekennzeichnet, dass** nach Prüfung der Bedingung des Alarmereignisses das mit dem Steuergerät des Fahrzeugmotors (CM) verbundene Steuergerät (I) die Gaspedalposition in Bezug auf die maximale Beschleunigungsposition und momentane Fahrzeuggeschwindigkeit hinsichtlich eines gegebenen Grenzwerts erfasst und wobei, wenn die erfasste Gaspedalposition im Vergleich zur Gaspedalposition bei Vollgas niedriger ist als 10 %, und wenn die momentane Geschwindigkeit des Fahrzeugs unter diesem Wert liegt, das Steuergerät (I) die besagte Elektronikeinheit (CM) des Motors anweist, die momentane Höchstgeschwindigkeit auf den besagten Grenzwert zu beschränken.

**20.** Elektronische Vorrichtung zur Überwachung und Verarbeitung von Informationen über das Fahrzeug im Allgemeinen gemäß Patentanspruch 19, **dadurch gekennzeichnet, dass** der Grenzwert der momentanen Höchstgeschwindigkeit 50 km/h beträgt.

**21.** Elektronische Vorrichtung zur Überwachung und Verarbeitung von Informationen über das Fahrzeug im Allgemeinen gemäß Patentanspruch 2, **dadurch gekennzeichnet, dass** sie abnehmbar am Fahrzeug befestigt ist, permanent am besagten Steuergerät des Fahrzeugmotors (CM) angeschlossen ist und durch die Zündungssteuerung aktiviert wird, und wobei die Motorzündung an die Erfassung und Prüfung der Informationen und der Bedingungen gemäß der vorstehenden Patentansprüche gebunden ist.

**Revendications**

**1.** Dispositif électronique pour la détection et l'élaboration de données concernant la composition de l'air à l'intérieur de l'habitacle de véhicules en général, ayant le but de prévenir et limiter les risques d'accidents de la route attribuable à l'utilisation d'alcool et/ou de ses substances associées **caractérisé en ce qu'**il comprend :

■ un ou plusieurs capteurs d'environnement (S1, ..., Sn) de la concentration correspondante ou titre dans l'air d'une ou plusieurs substances (X);
■ au moins une unité d'élaboration (UE) comparant les données obtenues des capteurs (S1, ..., Sn) avec des valeurs limites de référence prédéterminée,

où ladite unité d'élaboration (UE) actionne, directement ou indirectement, un signal d'évènement d'alarme conséquent à la comparaison positive des données dérivant des capteurs,
et où lesdits capteurs (S1, ..., Sn) sont réglés selon la substance certaine (X) en la changeant, sur un certain intervalle de temps (tf), par une valeur prédéfinie et connue de la concentration ou le titre (Xa) de ladite substance (X) injectée dans l'habitacle du véhicule, en produisant ainsi n vecteurs de valeurs de référence (S1r(t), ..., Snr(t)), où t varie de 0 à tf.

2. Dispositif électronique pour la détection et l'élaboration de données pour le véhicule en général, selon la revendication 1, **caractérisé en ce qu'**au moins une unité de contrôle ou interface (I) communiquant avec l'unité d'élaboration (UE) et interfacée à l'unité électronique du moteur du véhicule (CM), pour modifier les prestations des caractéristiques du véhicule en cas d'évènement d'alarme.

3. Dispositif électronique pour la détection et l'élaboration de données pour le véhicule en général, selon la revendication 2, **caractérisé en ce que** ladite unité de contrôle (I) est interfacée à ladite unité électronique du moteur du véhicule (CM), afin d'empêcher l'allumage du moteur du véhicule.

4. Dispositif électronique pour la détection et l'élaboration de données pour le véhicule en général, selon la revendication 2, **caractérisé en ce que** ladite unité de contrôle (I) est interfacée à ladite unité électronique du moteur du véhicule pour limiter la vitesse du véhicule quand il est actionné ou au début de l'allumage du moteur.

5. Dispositif électronique pour la détection et l'élaboration de données pour le véhicule en général, selon la revendication 1, **caractérisé en ce qu'**au moins une unité de contrôle (I), communiquant avec l'unité d'élaboration (UE) et interfacée à l'unité électronique du moteur du véhicule (CM) pour actionner des signalisations acoustiques et/ou lumineuses en cas d'événement d'alarme.

6. Dispositif électronique pour la détection et l'élaboration de données pour le véhicule en général, selon les revendications 1, 2, 3, 4, 5, **caractérisé en ce que** ladite substance (X) est alcool ou éthanol ou stupéfiant ou substances associées.

7. Dispositif électronique pour la détection et l'élaboration de données pour le véhicule en général, selon les revendications 1, 2, 3, 4, 5, **caractérisé en ce que** ladite substance (X) est oxygène.

8. Dispositif électronique pour la détection et l'élaboration de données pour le véhicule en général, selon les revendications 1, 2, 3, 4, 5, **caractérisé en ce que** ladite substance (X) est une substance stupéfiante et/ou une substance médicale et/ou une autre substance en dosage acceptable qui permet le conducteur de conduire le véhicule.

9. Dispositif électronique pour la détection et l'élaboration de données pour le véhicule en général, selon l'une quelconque des revendications précédentes, **caractérisé en ce que** lesdits capteurs (S1,..., Sn) sont du type à état solide, par exemple MEMS, ou cellules à combustible.

10. Dispositif électronique pour la détection et l'élaboration de données pour le véhicule en général, selon l'une quelconque des revendications précédentes, **caractérisé en ce que** lesdits capteurs (S1, ..., Sn) acquièrent lesdites données à intervalles de temps inférieurs à 180 secondes.

11. Dispositif électronique pour la détection et l'élaboration de données pour le véhicule en général, selon l'une quelconque des revendications précédentes, **caractérisé en ce qu'**il comprend deux ou plusieurs desdits capteurs (S1, ..., Sn) distribués dans l'habitacle du véhicule dans l'espace environnant la place du conducteur, à des distances et positions déterminées par rapport à la place du conducteur.

12. Dispositif électronique pour la détection et l'élaboration de données pour le véhicule en général, selon l'une quelconque des revendications précédentes, **caractérisé en ce que** ledit intervalle de temps (tf) est proportionnel à la dimension de l'habitacle du véhicule, au titre de la/des substance/s à détecter et à la disposition spatiale des capteurs.

13. Dispositif électronique pour la détection et l'élaboration de données pour le véhicule en général, selon la revendication 12, **caractérisé en ce que** ledit intervalle de temps (tf) est inférieur à 180 secondes.

14. Dispositif électronique pour la détection et l'élaboration de données pour le véhicule en général, selon les revendications 12, 13, **caractérisé en ce que** :

■ en conditions de fonctionnement normal, lesdits capteurs (S1, ..., Sn) détectent des valeurs de concentration de ladite substance (X), en générant une pluralité de vecteurs ayant des valeurs élevées (S1(t), ..., Sn(t)) ;
■ ladite unité d'élaboration (UE) réunit lesdits vecteurs de valeurs élevées (S1(t), ..., Sn(t)) et lesdits vecteurs de valeurs de référence (S1r(t), ..., Snr(t)) et effectue n convolutions mutuelles entre chaque valeur détectée (Si(t)) et la correspondante valeur de référence (Sir(t)), en générant n vecteurs de valeurs (S1C(t), ..., SnC(t)), où t varie de 0 à tf et où

$$SiC\,(nt) = \sum_{k=0}^{n} Si(kt) * Sir((n-k)t)$$

avec i qui varie de 1 à n ;
ladite unité d'élaboration (UE) effectue une intégration dans le temps de chacune desdites valeurs SiC(t), en générant n valeurs (Ai), une pour chaque capteur Si, où

$$Ai = \sum_{n=0}^{n-max} SiC\,(nt)$$

avec i qui varie de 1 à n ;
ladite unité d'élaboration (UE) compare lesdites n valeurs (Ai) avec les n valeurs correspondantes (Ari) de référence déterminées expérimentalement,
et où l'évènement d'alarme ou de blocage immédiat ou différé du moteur se vérifie au cas où ladite comparaison résulterait positive pour un pourcentage déterminé desdites valeurs (Ai).

15. Dispositif électronique pour la détection et l'élaboration de données pour le véhicule en général, selon la revendication 14, **caractérisé en ce que** chacune desdites valeurs de référence (Ari) est égale à 30% de la valeur maximum possible de la correspondante valeur (Ai).

16. Dispositif électronique pour la détection et l'élaboration de données pour le véhicule en général, selon les revendications 14, 15, **caractérisé en ce que** ledit évènement d'alarme ou de blocage immédiat ou différé du moteur se vérifie au cas où ladite comparaison résulterait positive pour un pourcentage plus grand ou égal à 50% desdites valeurs (Ai).

17. Dispositif électronique pour la détection et l'élaboration de données pour le véhicule en général, selon la revendication 16, **caractérisé en ce que** ladite comparaison est positive si :

■ Ai < Ari, pour substances (X) dont la concentration doit être supérieurement limitée, comme éthanol ou substances associées ;
■ Ai > Ari, pour substances (X) dont la concentration doit être inférieurement limitée, comme oxygène.

18. Dispositif électronique pour la détection et l'élaboration de données pour le véhicule en général, selon la revendication 1, **caractérisé en ce que** ladite unité d'élaboration (UE) multiplie chacune desdites valeurs détectées du vecteur (S1(t), ..., Sn(t)) par une valeur constante (Kn), où :

■ Kn = 1, pour alcool ou éthanol ;
■ Valeur absolue de Kn > 1, pour substances plus dangereuses que l'alcool ;
■ Kn < 0, pour substances dont la concentration doit être inférieurement limitée.

19. Dispositif électronique pour la détection et l'élaboration de données pour le véhicule en général, selon la revendication 4, **caractérisé en ce que** si la condition d'événement d'alarme se vérifie, alors ladite unité de contrôle (I), s'interfaçant avec ladite unité électronique du moteur (CM), détecte la position de l'accélérateur par rapport à la position d'accélération maximum et la vitesse instantanée de marche du véhicule est inférieure à ladite valeur limite, et où, si ladite position détectée de la pédale d'accélérateur est inférieure à 10% comparée à la position de pédale correspondant à l'accélération maximale et si la vitesse instantanée du véhicule est inférieure à ladite valeur, alors l'unité de contrôle (I) communique à ladite unité électronique (CM) du moteur de limiter la vitesse instantanée di marche maximum à ladite valeur limite.

20. Dispositif électronique pour la détection et l'élaboration de données pour le véhicule en général, selon la revendication 19, **caractérisé en ce que** ladite valeur limite de la vitesse instantanée maximum est 50 km/h.

21. Dispositif électronique pour la détection et l'élaboration de données pour le véhicule en général, selon la revendication 2, **caractérisé en ce qu'**il est limité de manière amovible au véhicule, relié de manière permanente à ladite unité électronique (CM) et actionné par l'introduction de la clé d'allumage du véhicule et où l'allumage du moteur est conditionné par la détection et la vérification des données et des conditions selon les revendications précédentes.

Fig. 1

**EP 2 183 124 B1**